# EUROPEAN PATENT APPLICATION

(11) **EP 3 666 332 A1**
(43) Date of publication of application: **17.06.2020**
(21) Application number: 18211779.6
(22) Date of filing: 11.12.2018
(51) Int. Cl.: A61N 5/06

(54) **DEVICE FOR TREATING TISSUE**

(71) Applicant: Koninklijke Philips N.V., 5656 AG Eindhoven (NL)
(72) Inventor: Uzunbajakava, Natallia Eduarda, 5656 AE Eindhoven (NL); Johnson, Mark Thomas, 5656 AE Eindhoven (NL); Varghese, Babu, 5656 AE Eindhoven (NL)
(74) Representative: de Haan, Poul Erik

(57) **Abstract**

There is provided a device (100) for treating tissue (102). The device (100) comprises a light source (104) configured to emit light (106) to illuminate the tissue (102). The light source (104) is configured to alternate between emitting light of a first wavelength to stimulate at least partial photoconversion of a protein in at least part of the tissue (102) and emitting light of a second wavelength to at least partially reverse the at least partial photoconversion of the protein in the at least part of the tissue (102). The first wavelength and the second wavelength are different.

## Description

### FIELD OF THE INVENTION

The disclosure relates to a device and system for treating tissue and a method for treating tissue.

### BACKGROUND OF THE INVENTION

Light therapy (or phototherapy) has been used in the treatment of tissue for many years. In the past decade, the use of low-level laser therapy (LLLT) or photobiomodulation therapy (PBMT) has become increasing popular techniques in this field. These techniques apply low-level (or low-power) lasers or light emitting diodes (LEDs) to tissue in order to treat the tissue. For example, the techniques have been found to provide a wide range of benefits, such as improving tissue repair, reducing pain and/or inflammation, treating cutaneous disorders, and so on. The techniques rely on the low-level of light applied to the tissue inducing photochemical and photobiological reactions in the tissue and it is these reactions that provide these benefits.

Recently, new knowledge has emerged on the existence of photosensitive receptors in tissue. Generally, these photosensitive receptors that exist in tissue are proteins. An example of such a protein is an opsin. The proteins are present in various types of tissue, including the skin, hair follicles, blood vessels, brain, fat, etc. When stimulated by light, the proteins have been seen to provide useful medical benefits. For example, different types of opsins stimulated by ultraviolet (UV) light have been shown to participate in the production of a melanin pigment, improve vasorelaxation and/or vasodilation, maintain the hair growth phase, improve fat metabolism, aid in the differentiation of epidermal keratinocytes, and so on. US 2011/0125077 discloses an example of a technique that uses optical stimulation of a target tissue to activate light-sensitive proteins, such as opsins.

However, while there are useful benefits that arise from stimulating proteins with light, the existing techniques have limitations. In particular, once the proteins have been converted into a stable photoproduct, further repeated irradiation using the same wavelength results in desensitization to light and therefore does not result in further therapeutic benefits.

### SUMMARY OF THE INVENTION

As noted above, a limitation with existing devices is that repeated irradiation using the same wavelength results in proteins being desensitized to light and thus further therapeutic benefits are not possible. It would thus be valuable to have an improvement to address this limitation.

Therefore, according to a first aspect, there is provided a device for treating tissue. The device comprises a light source configured to emit light to illuminate tissue. The light source is configured to alternate between emitting light of a first wavelength to stimulate at least partial photoconversion of a protein in at least part of the tissue and emitting light of a second wavelength to at least partially reverse the at least partial photoconversion of the protein in the at least part of the tissue. The first wavelength and the second wavelength are different.

In some embodiments, the light source may be configured to alternate between emitting the light of the first wavelength to stimulate partial photoconversion of the protein in at least part of the tissue and emitting the light of the second wavelength to at least partially reverse the partial photoconversion of the protein in the at least part of the tissue.

In some embodiments, the light source may be configured to emit the light of the first wavelength for a predefined time period to stimulate partial photoconversion of the protein in at least part of the tissue. In some embodiments, the light source may be configured to emit the light of the first wavelength at a predefined intensity to stimulate partial photoconversion of the protein in at least part of the tissue.

In some embodiments, the light source may be configured to alternate between emitting the light of the first wavelength to stimulate photoconversion of the protein in a first part of the tissue and emitting the light of the second wavelength to at least partially reverse the photoconversion of the protein in the first part of the tissue.

In some embodiments, the light source may be configured to emit the light of the first wavelength to illuminate the first part of the tissue to stimulate photoconversion of the protein in the first part of the tissue and emit the light of the second wavelength to illuminate a second part of the tissue to at least partially reverse the photoconversion of the protein in the first part of the tissue.

In some embodiments, the light source may comprise a first light source configured to emit the light of the first wavelength to illuminate the first part of the tissue and a second light source configured to emit the light of the second wavelength to illuminate the second part of the tissue. In some embodiments, the light source may comprise a filter configured to pass the light of the first wavelength to illuminate the first part of the tissue and pass the light of the second wavelength to illuminate the second part of the tissue.

In some embodiments, the light source may be configured to alternate between (a) emitting the light of the first wavelength to stimulate photoconversion of the protein in a first part of the tissue and emitting the light of the second wavelength to at least partially reverse the photoconversion of the protein in the first part of the tissue and (b) emitting the light of the first wavelength to stimulate photoconversion of the protein in a second part of the tissue and emitting the light of the second wavelength to at least partially reverse the photoconversion of the protein in the second part of the tissue.

In some embodiments, the light source may be configured to emit the light of the first wavelength to illuminate the first part of the tissue to stimulate photoconversion of the protein in the first part of the tissue and emit the light of the second wavelength to illuminate the second part of the tissue to at least partially reverse the photoconversion of the protein in the first part of the tissue.

In some embodiments, the light source may be configured to emit the light of the first wavelength to illuminate the second part of the tissue to stimulate photoconversion of the protein in the second part of the tissue and emit the light of the second wavelength to illuminate the first part of the tissue to at least partially reverse the photoconversion of the protein in the second part of the tissue.

In some embodiments, the device may comprise a lens configured to focus the light of the first wavelength at a first predefined depth in the tissue and/or focus the light of the second wavelength at a second predefined depth in the tissue. In some embodiments, the second predefined depth may be the same as the first predefined depth or different to the first predefined depth.

According to a second aspect, there is provided a system comprising the device as claimed in any of the preceding claims and a processor. In some embodiments, the processor may be configured to acquire an indication of a photoconversion state of the protein and control the light source to alternate between emitting the light of the first wavelength and the light of the second wavelength according to the photoconversion state of the protein.

According to a third aspect, there is provided a method for treating tissue. The method comprises alternating between emitting light of a first wavelength to stimulate at least partial photoconversion of a protein in at least part of the tissue and emitting light of a second wavelength to at least partially reverse the at least partial photoconversion of the protein in the at least part of the tissue. The first wavelength and the second wavelength are different.

According to a fourth aspect, there is provided a computer program product comprising a computer readable medium. The computer readable medium has computer readable code embodied therein. The computer readable code is configured such that, on execution by a suitable computer or processor, the computer or processor is caused to perform the method described earlier.

According to the aspects and embodiments described above, the limitations of existing techniques are addressed. In particular, according to the above-described aspects and embodiments, in addition to the light source being configured to emit light of a first wavelength to stimulate at least partial photoconversion of a protein in at least part of the tissue, the light source is also configured to emit light of second wavelength that is different to the first wavelength. The emission of the light of the second wavelength at least partially reverses the at least partial photoconversion of the protein in the at least part of the tissue. In this way, the proteins can be brought back to (or close to) their original state. Moreover, the light source is configured to alternate between emitting the light of the first wavelength and the light of the second wavelength, which means that the protein can be repeatedly brought into (or close to) a stable photoproduct and back to (or close to) their original state. This means that desensitization of the protein can be reversed, which enables continued or further therapeutic benefits to be provided. There is thus provided an improved device and system for treating tissue and an improved method for treating tissue.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a simplified schematic illustration of a device according to an embodiment;
Fig. 2 is an illustration of absorption spectra for a photoconvertable protein;
Fig. 3 is an illustration of absorption spectra for a photoconvertable protein;
Fig. 4 is an illustration of absorption spectra for a photoconvertable protein;
Fig. 5 is a simplified schematic illustration of light emitted by a device to illuminate tissue according to an embodiment;
Fig. 6 is a simplified schematic illustration of light emitted by a device to illuminate tissue according to an embodiment;
Fig. 7 is a simplified schematic illustration of light emitted by a device to illuminate tissue according to an embodiment;
Figs. 8(a), (b) and (c) are simplified schematic illustrations of example lenses according to an embodiment; and
Fig. 9 is a flow chart illustrating a method according to an embodiment.

### DETAILED DESCRIPTION OF THE EMBODIMENTS

As noted above, there is provided herein an improved device and system for treating tissue and an improved method for treating tissue.

In some embodiments, the device described herein can be a wearable device (e.g. a wearable patch). That is, in some embodiments, the device may be configured to be worn by a subject for treating tissue of the subject according to some embodiments. In some embodiments, the device described herein may be a handheld device. That is, in some embodiments, the device may be configured to be held by a user (e.g. the subject or another user) for treating tissue of the subject according to some embodiments. In some embodiments, the device described herein can be an insertable or implantable device. That is, in some embodiments, the device may be configured to be inserted into the tissue (or the subject) or implanted into the tissue (or the subject) for treating tissue of the subject. In some embodiments, the device described herein may be configured to be placed on a surface in the environment of the subject (e.g. on top of a table or bed), for treating tissue of the subject.

The tissue referred to herein may comprise any type of tissue. For example, the tissue may comprise epithelial tissue such as the skin (e.g. the epidermis of the skin, the cells of the skin, hair follicles, or similar) or the lining of hollow organs (e.g. the lining of gastrointestinal tract organs or any other hollow organs), muscle tissue (e.g. cardiac muscle tissue, skeletal muscle, or any other muscle tissue), connective tissue (e.g. fat, bone, tendons, or any other connective tissue), nervous tissue (e.g. the brain, spinal cord, nerves, or any other nervous tissue), blood vessels, or any other tissue, or any combination of tissue.

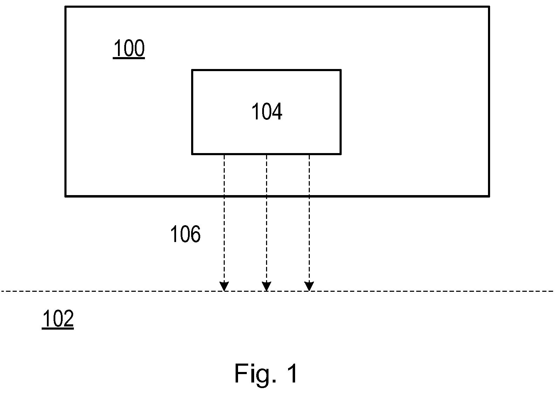

Fig. 1 illustrates a device 100 for treating tissue 102 according to an embodiment. As illustrated in Fig. 1, the device 100 comprises a light source 104 configured to emit light to illuminate the tissue 102. The light source 104 is configured to alternate between emitting light of a first wavelength to stimulate at least partial photoconversion of a protein in at least part of the tissue 102 and emitting light of a second wavelength to at least partially reverse the at least partial photoconversion of the protein in the at least part of the tissue 102. The first wavelength and the second wavelength are different.

The first wavelength and the second wavelength referred to herein can be wavelengths in a range from 300 to 1000 nm. In some embodiments, the first wavelength referred to herein may be a wavelength in a range from 325 to 555 nm. In some embodiments, the first wavelength referred to herein may be a wavelength in a range from 400 to 555 nm, for example a wavelength in a range from 410 to 510 nm. In some embodiments, the first wavelength referred to herein may be a wavelength in a range from 325 to 425 nm, for example a wavelength in a range from 350 to 410 nm. In some embodiments, the second wavelength referred to herein may be a wavelength in a range from 470 to 600 nm, for example a wavelength in a range from 510 to 560 nm. The second wavelength may be a wavelength that is longer than the peak absorption (maximum absorption) wavelength of the photoconverted protein. The photoconverted protein may also be referred to herein as a photoproduct protein. A person skilled in the art will be able to identify first and second wavelengths for any given photoconvertable protein.

The light source 104 referred to herein can be any type of light source. The light source 104 may, for example, comprise one or more coherent light sources and/or one or more incoherent light sources. An example of a coherent light source includes, but is not limited to, a laser. Examples of incoherent light sources include, but are not limited to, a light emitting diodes (LED) and an organic light emitting diode (OLED). The light source may comprise any one or more of these light sources and/or any other type of light source. The light source may also comprise any combination of light sources. The light source 104 may comprise a single light source or a plurality of light sources, e.g. an array of light sources.

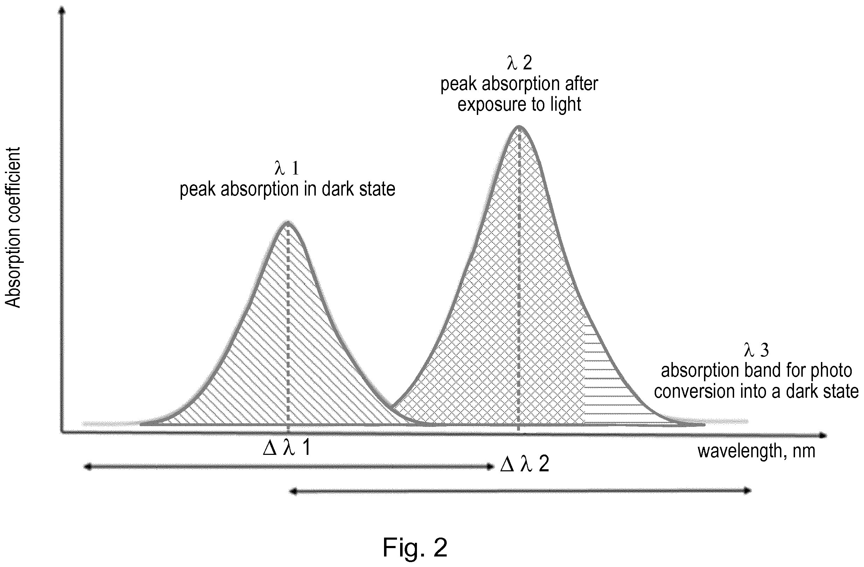

Herein, the protein in at least part of the tissue 102 is a photoconvertable protein. That is, a protein that undergoes photoconversion from a native (or dark) state to a photoproduct protein. The protein referred to herein can be any photoconvertable protein, such as an opsin, or any other photoconvertable protein, e.g. containing G-protein Coupled Receptors (GPCRs). The photoconversion of the protein may be a conformational conversion of the protein. That is, the absorption of light by the protein in its native (or dark) state may change a conformation of the protein. This change in conformation may, for example, initiate physiological reactions in the tissue 102. The change in conformation converts the protein into the photoproduct protein. The photoproduct protein may be a stable photoproduct. That is, the photoproduct protein may be resistant to change when exposed to light. The absorption properties of the photoproduct protein can be different to those of the original photoconvertable protein, e.g. the absorption properties of the photoproduct protein may be shifted towards longer wavelengths and/or may have a higher absorption value compared to the original photoconvertable protein. This is illustrated in Figs. 2, 3 and 4.

Fig. 2 is an illustration of absorption spectra for a photoconvertable protein. More specifically, Fig. 2 is an illustration of an absorption coefficient of a photoconvertable protein as a function of a wavelength of light.

In Fig. 2, λ1 corresponds to the wavelength at which a peak absorption (maximum absorption) occurs in the protein when in its native (or dark) state, i.e. prior to photoconversion to a photoproduct protein. λ2 corresponds to the wavelength at which a peak absorption (maximum absorption) occurs in the protein after the protein has been exposed to light, i.e. once the protein has been photoconverted into a photoproduct protein. In Fig. 2, λ3 corresponds to the wavelength selected for at least partially reversing the photoconversion of the protein in the at least part of the tissue 102, i.e. for at least partially photoconverting the photoproduct protein back into the native (or dark) state of the photoconvertable protein. That is, λ3 corresponds to the second wavelength referred to herein.

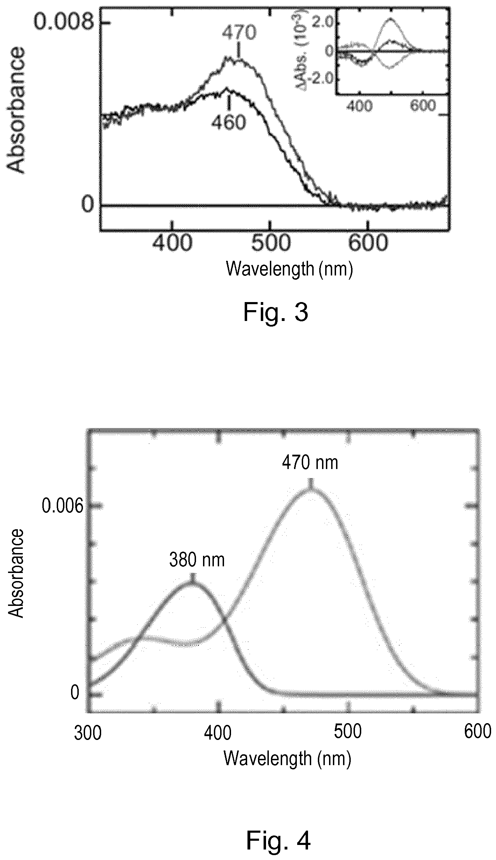

For some photoconvertable proteins, λ1 and λ2 may be closer to each other. In some cases, the absorption spectra and the peak absorption (maximum absorption) of the native (or dark) state photoconvertable proteins and their photoproduct proteins may overlap. An example of such a photoconvertable protein is illustrated in Fig. 3.

Fig. 3 is an illustration of absorption spectra for a photoconvertable protein. More specifically, Fig. 3 is an illustration of absorbance of a photoconvertable protein as a function of a wavelength of light. In the example illustrated in Fig. 3, the photoconvertable protein is a human opsin called encephalopsin or panopsin, which can also be referred to as opsin-3 or OPN3. OPN3 is present in various tissues, such as in the brain, eyes, liver, inner root sheath of the hair follicles epidermis, dermal fibroblasts, and human epidermal melanocytes.

As illustrated in Fig. 3, the wavelength at which a peak absorption (maximum absorption) occurs in OPN3 when in its native (or dark) state, i.e. prior to photoconversion to a photoproduct protein, is 460 nm (i.e. λ1 = 460 nm). The full width half maximum (FWHM) of this peak is approximately 40 nm. As also illustrated in Fig. 3, the wavelength at which a peak absorption (maximum absorption) occurs in OPN3 after exposure of OPN3 to blue light, i.e. once OPN3 has been photoconverted into a photoproduct protein, is 470 nm (i.e. λ2 = 470 nm). The full width half maximum (FWHM) of this peak is approximately 50 nm. OPN3 absorbs blue light and thus, when exposed to blue light, OPN3 photoconverts into a photoproduct protein.

The blue-absorbing photoproduct of OPN3 (with its maximum absorption at 470 nm) is stable in the absence of light and reverts back into its native (or dark) blue-absorbing state by the subsequent illumination with orange light (long-pass filter, 50% cut off at 560 nm). Thus, a wavelength of 560 nm or above is equivalent to λ3 of Fig. 2. That is, a wavelength of 560 nm or above corresponds to the second wavelength referred to herein, which is selected for at least partially reversing the photoconversion of OPN3 in the at least part of the tissue 102.

Fig. 4 is an illustration of absorption spectra for a photoconvertable protein. More specifically, Fig. 4 is an illustration of absorbance of a photoconvertable protein as a function of a wavelength of light. In the example illustrated in Fig. 4, the photoconvertable protein is a human opsin called neuropsin, which can also be referred to as opsin-5 or OPN5. OPN5 is present in various tissues, such as in the testis, epididymis, epidermis basal layer, human epidermal melanocytes, and epidermal keratinocytes.

As illustrated in Fig. 4, the wavelength at which a peak absorption (maximum absorption) occurs in OPN5 when in its native (or dark) state, i.e. prior to photoconversion to a photoproduct protein, is 380 nm (i.e. λ1 = 380 nm). The full width half maximum (FWHM) of this peak is approximately 20 nm. As also illustrated in Fig. 4, the wavelength at which a peak absorption (maximum absorption) occurs in OPN5 after exposure of OPN5 to ultraviolet (UV) light, i.e. once OPN5 has been photoconverted into a photoproduct protein, is 470 nm (i.e. λ2 = 470 nm). The full width half maximum (FWHM) of this peak is approximately 50 nm. OPN5 absorbs UV light and thus, when exposed to UV light, OPN5 photoconverts into a photoproduct protein.

The blue-absorbing photoproduct of OPN5 (with its maximum absorption at 470 nm) is stable in the absence of light and reverts back into its native (or dark) UV-absorbing state by the subsequent illumination with orange light (long-pass filter, 50% cut off at 520 nm). Thus, a wavelength of 520 nm or above is equivalent to λ3 of Fig. 2. That is, a wavelength of 520 nm or above corresponds to the second wavelength referred to herein, which is selected for at least partially reversing the photoconversion of OPN5 in the at least part of the tissue 102.

A summary of the spectral properties of OPN3 and OPN5 is provided in the following table:

| **Protein (opsin)** | **Maximum absorption (dark state)** | **FWHM (dark state)** | **Maximum absorption (photo-product state)** | **FWHM (photo-product state)** | **Wavelength to reverse photoconversion** |
|---|---|---|---|---|---|
| **OPN3** | 460 nm | +/- 50 nm | 470 nm | +/- 50 nm | Orange O56 glass cutoff longpass filter with 50% T at 560 nm |
| **OPN5** | 380 nm | +/- 30 nm | 470 nm | +/- 40 nm | >480 nm; e.g. 520 nm |

In some embodiments, for OPN3, the first wavelength may be a wavelength in a range from 325 to 555 nm, for example a wavelength in a range from 400 to 555 nm, for example a wavelength in a range from 410 to 510 nm. In some embodiments, for OPN5, the first wavelength may be a wavelength in a range from 325 to 425 nm, for example a wavelength in a range from 350 to 410 nm. In some embodiments, for OPN3 and OPN5, the second wavelength may be a wavelength in a range from 470 to 600 nm, for example a wavelength in a range from 510 to 560 nm.

Although only OPN3 and OPN5 have been described as examples, it will be understood that other opsins (such as those outside of visual tissue, e.g. OPN2, OPN1, OPN4) and other photoconvertable proteins will exhibit a similar behavior. That is, other opsins will also behave as photoconvertable proteins in the manner described herein. The disclosure thus applies to any photoconvertable protein.

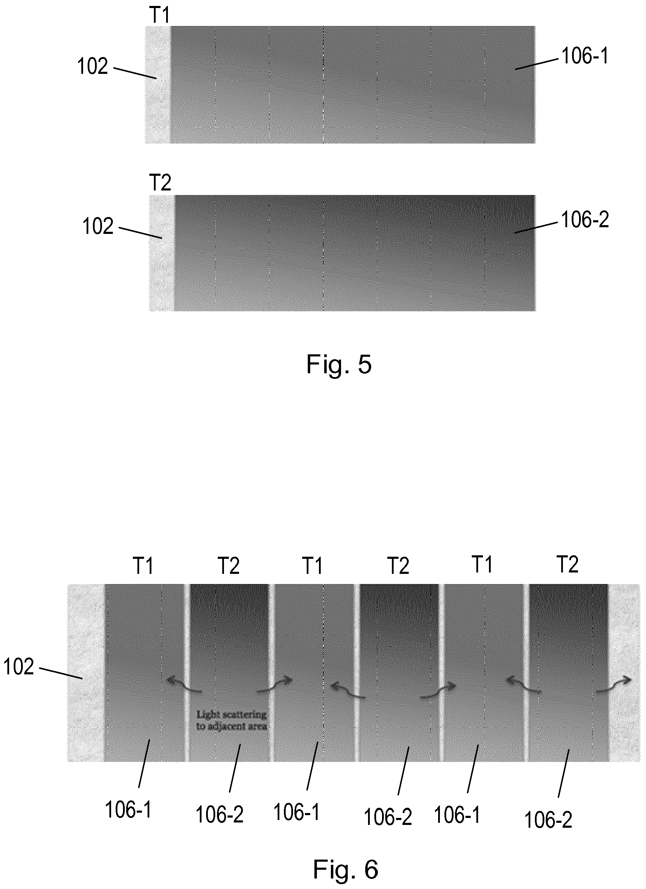

Thus, as described earlier, the light source 104 of the device 100 described herein is configured to alternate between emitting light of a first wavelength to stimulate at least partial photoconversion of a protein in at least part of the tissue 102 and emitting light of a second wavelength to at least partially reverse the at least partial photoconversion of the protein in the at least part of the tissue 102. The light source 104 of the device 100 can be configured to alternate between emitting light of the first wavelength and emitting light of the second wavelength in various ways. Some of these ways will now be described with reference to Figs. 5-7.

In some embodiments, the light source 104 of the device 100 may be configured to alternate between emitting light of the first wavelength 106-1 to stimulate partial photoconversion of the protein in the at least part of the tissue 102 and emitting light of the second wavelength 106-2 to at least partially reverse the partial photoconversion of the protein in the at least part of the tissue 102. In these embodiments, the light source 104 of the device 100 may be configured to emit the light of the first wavelength 106-1 and the light of the second wavelength to illuminate the same part of the tissue 102 or different part of the tissue. As the light source 104 of the device 100 in these embodiments is configured to emit light of the first wavelength 106-1 to stimulate only partial photoconversion of the protein in at least part of the tissue 102, the photoconversion of the protein never actually completes.

Fig. 5 is a simplified schematic illustration of light 106 emitted by the device 100 described earlier to illuminate tissue 102 according to such an embodiment. In the embodiment illustrated in Fig. 5, the light source 104 of the device 100 is configured to emit the light of the first wavelength 106-1 for a predefined time period to stimulate partial photoconversion of the protein in the at least part of the tissue 102. The predefined time period may be referred to as a pulse duration. In some embodiments, the pulse duration can be defined according to a pulsing frequency and may, for example, be defined based on one or more biological processes, such as any one or more of the protein photoconversion itself, calcium ion dynamics, and dynamics of ion channels (where the latter two participate in transduction of the light initiated by photoconvertable proteins). Examples of a pulsing frequency that can be used include, but are not limited to 1000Hz (e.g. for a pulse duration in the milliseconds range), 1 Hz (e.g. for a pulse duration in the seconds range), or 0.01 Hz (e.g. for a pulse duration in the minutes range). Thus, in some embodiments, the light source 104 of the device 100 may be configured to cease emitting the light of the first wavelength 106-1 upon expiry of the predefined time period. In this way, the light source 104 of the device 100 can be configured to cease emitting the light of the first wavelength 106-1 before the photoconversion of the protein completes. The predefined time period can be defined based on the time taken for complete photoconversion of the protein to occur. In particular, the predefined time period can be set to be shorter than the time taken for complete photoconversion of the protein to occur. The light source 104 of the device 100 may be configured to emit the light of the second wavelength 106-2 before, on, or after expiry of the predefined time period.

As illustrated in Fig. 5, in some embodiments, the light source 104 of the device 100 may be configured to emit light of the first wavelength 106-1 at a first time T1 and to emit light of the second wavelength 106-2 at a second time T2. The first time T1 and the second time T2 are different. Thus, in some embodiments, the light source 104 of the device 100 may be configured to emit light of the first wavelength 106-1 and to emit light of the second wavelength 106-2 consecutively or sequentially. In some embodiments, the light source 104 of the device 100 may be configured to pulse the emission of the light of the first wavelength 106-1 and the light of the second wavelength 106-2, e.g. according to the predefined time period (or pulse duration).

In another example (not illustrated), alternatively or in addition to the predefined time period, in some embodiments, the light source 104 of the device 100 may be configured to emit the light of the first wavelength 106-1 at a predefined intensity to stimulate partial photoconversion of the protein in the at least part of the tissue 102. For example, the predefined intensity may be set so as to only stimulate partial photoconversion of the protein in the at least part of the tissue 102. Thus, in some embodiments, the light source 104 of the device 100 may be configured to emit the light of the first wavelength 106-1 at an intensity that does not allow complete photoconversion of the protein. In some embodiments, the predefined intensity can be an intensity in a range from 1 to 200 mW/cm² or an intensity up to 500 mW/cm². In some embodiments, the predefined intensity may be set based on the predefined time period mentioned earlier, or vice versa.

In this way, in some embodiments, the light dosage provided to the at least part of the tissue 102 by the light source 104 of the device 100 can be restricted (e.g. by limiting the time for which and/or the intensity with which the light source 104 is configured to emit the light of the first wavelength 106-1) to avoid complete photoconversion of the protein in the at least part of the tissue 102. In some embodiments, the light dosage provided to the at least part of the tissue 102 by the light source 104 of the device 100 may be in a range from 1J/cm² to 100 J/cm².

The embodiments where the light source 104 of the device 100 is configured to emit light of the first wavelength 106-1 to stimulate only partial photoconversion of the protein in the at least part of the tissue 102, such that the photoconversion of the protein never actually completes, can be advantageous. In particular, it can be that proteins for which complete photoconversion occurs mask off proteins in deeper layers of the tissue. Also, due to the limited depth in the tissue to which light can penetrate, there will be faster protein conversion closer to the skin surface. This means that the treatment may be restricted to superficial layers of tissue and it may not be possible for the treatment to penetrate to deeper layers that are masked. It can also be difficult to reverse complete photoconversion. Thus, by ensuring that photoconversion of proteins never actually completes, the masking that may otherwise hinder the treatment of deeper layers of tissue can be avoided or the effect of masking can at least be reduced and a more effective treatment is then possible.

Fig. 6 is a simplified schematic illustration of light 106 emitted by the device 100 described earlier to illuminate tissue 102 according to another embodiment. In the embodiment illustrated in Fig. 6, the light source 104 of the device 100 is configured to alternate between emitting the light of the first wavelength 106-1 to stimulate photoconversion of the protein in a first part of the tissue 102 and emitting the light of the second wavelength 106-2 to at least partially reverse the photoconversion of the protein in the first part of the tissue 102. Thus, in this embodiments, complete photoconversion of the protein is allowed, which can provide treatment advantages.

In some embodiments, as illustrated in Fig. 6, the light source 104 may be configured to emit the light of the first wavelength 106-1 to illuminate the first part of the tissue 102 to stimulate photoconversion of the protein in the first part of the tissue 102 and emit the light of the second wavelength 106-2 to illuminate a second part of the tissue 102 to at least partially reverse the photoconversion of the protein in the first part of the tissue 102. The first part of the tissue 102 and the second part of the tissue 102 can be different. For example, the first part of the tissue 102 may be one half of the tissue and the second part of the tissue 102 may be the other half of the tissue 102. The first part of the tissue 102 may be adjacent to (e.g. next to or adjoining) the second part of the tissue 102 according to some embodiments. Thus, in some embodiments, a patterned illumination can be provided by illuminating different parts of the tissue 102 with light of different wavelengths. In some embodiments, for example, the illumination may be in the form of a chess board.

The emission of the light of the first wavelength 106-1 to illuminate the first part of the tissue 102 may at least partially stimulate photoconversion of the protein in the second part of the tissue 102 due to the light of the first wavelength 106-1 being scattered by the tissue 102 into the second part of the tissue 102. In general, as scattering spreads the light, the deeper it penetrates. As such, there may be a progressively widening photoconversion at greater depths. This results in a photoconversion of the lower reaches of the tissue 102, which would otherwise be potentially masked off for direct illumination. As illustrated in Fig. 6, the emission of the light of the second wavelength 106-2 to illuminate the second part of the tissue 102 may at least partially reverse the photoconversion of the protein in the first part of the tissue 102 due to the light of the second wavelength 106-2 being scattered by the tissue 102 into the first part of the tissue 102.

In some embodiments, as illustrated in Fig. 6, the light source 104 of the device 100 may be configured to emit light of the first wavelength 106-1 at a first time T1 and to emit light of the second wavelength 106-2 at a second time T2. The first time T1 and the second time T2 can be different. Thus, in some embodiments, the light source 104 of the device 100 may be configured to emit light of the first wavelength 106-1 and to emit light of the second wavelength 106-2 sequentially. In this way, a sequential illumination can be provided. In some embodiments, a patterned and sequential illumination can be provided. The patterned illumination means that only part of the tissue 102 is illuminated with the light of the first wavelength 106-1, such that photoconversion only occurs in that part of the tissue 102 and not in other parts of the tissue 102. That is, a proportion of the tissue is free from photoconversion. In particular, the proteins in the non-illuminated parts of the tissue 102 are not photoconverted. In this way, complete photoconversion can be allowed in some parts of the tissue 102, while the effect of masking is reduced since photoconversion does not occur at all in other parts of the tissue 102.

Although not illustrated, in some embodiments, the light source 104 of the device 100 may comprise a first light source configured to emit the light of the first wavelength 106-1 to illuminate the first part of the tissue 102 and a second light source configured to emit the light of the second wavelength 106-2 to illuminate the second part of the tissue 102. The first light source and the second light source may be different light sources. In this way, an array type illumination light source is provided. Thus, in some embodiments, different light sources may be configured to emit light to illuminate different parts of the tissue 102.

Although also not illustrated, in other embodiments, the light source 104 of the device 100 may comprise a filter. In these embodiments, the filter can be configured to pass the light of the first wavelength 106-1 to illuminate the first part of the tissue 102 and pass the light of the second wavelength 106-2 to illuminate the second part of the tissue 102. In some embodiments, the filter may comprise an illumination mask. For example, the filter may comprise one or more color filters. In some of these embodiments, the light source 104 of the device 100 may be a continuous light source.

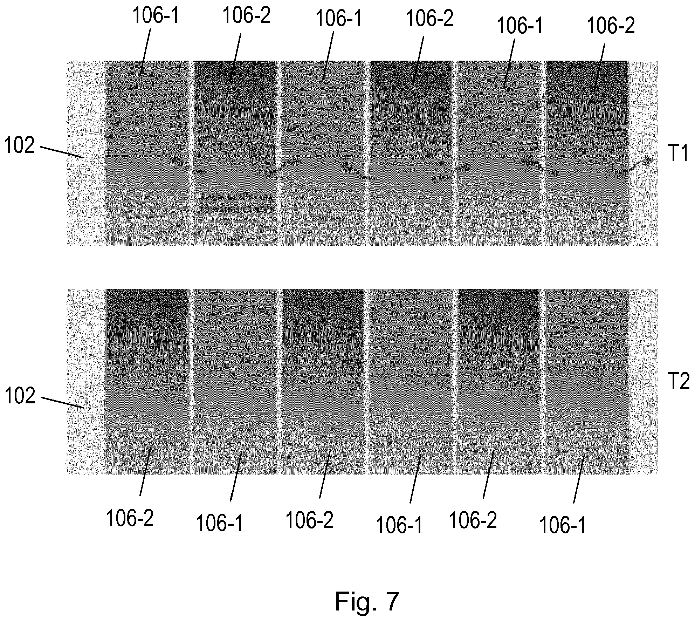

Fig. 7 is a simplified schematic illustration of light 106 emitted by the device 100 described earlier to illuminate tissue 102 according to an embodiment. In the embodiment illustrated in Fig. 7, the light source 104 of the device 100 is configured to alternate between (a) emitting the light of the first wavelength to stimulate photoconversion of the protein in a first part of the tissue and emitting the light of the second wavelength to at least partially reverse the photoconversion of the protein in the first part of the tissue and (b) emitting the light of the first wavelength to stimulate photoconversion of the protein in a second part of the tissue and emitting the light of the second wavelength to at least partially reverse the photoconversion of the protein in the second part of the tissue.

Thus, as illustrated in Fig. 7, in some embodiments, the light source 104 of the device 100 may be configured to emit the light of the first wavelength 106-1 to stimulate photoconversion of the protein in a first part of the tissue 102 and emit the light of the second wavelength 106-2 to at least partially reverse the photoconversion of the protein in the first part of the tissue 102 at a first time T1. In these embodiments, as illustrated in Fig. 7, the light source 104 of the device 100 may also configured to emit the light of the first wavelength 106-1 to stimulate photoconversion of the protein in a second part of the tissue 102 and the light of the second wavelength 106-2 to at least partially reverse the photoconversion of the protein in the second part of the tissue 102 at a second time T2. The first time T1 and the second time T2 can be different.

In some embodiments, the light source 104 of the device 100 can be configured to emit the light of the first wavelength 106-1 to illuminate the first part of the tissue 102 to stimulate photoconversion of the protein in the first part of the tissue 102 and emit the light of the second wavelength 106-2 to illuminate the second part of the tissue 102 to at least partially reverse the photoconversion of the protein in the first part of the tissue 102. In some embodiments, the light source 104 of the device 100 can be configured to emit the light of the first wavelength 106-1 to illuminate the second part of the tissue 102 to stimulate photoconversion of the protein in the second part of the tissue 102 and emit the light of the second wavelength 106-2 to illuminate the first part of the tissue 102 to at least partially reverse the photoconversion of the protein in the second part of the tissue 102.

Although not illustrated, in some embodiments (such as those illustrated in Figs. 6 and 7), the device 100 may comprises a lens. The lens can be configured to focus the light of the first wavelength 106-1 at a first predefined depth in the tissue 102. Alternatively or in addition, in some embodiments (such as those illustrated in Figs. 6 and 7), the lens can be configured to focus the light of the second wavelength 106-2 at a second predefined depth in the tissue. The second predefined depth may be the same as the first predefined depth or different to the first predefined depth. In some embodiments, the second predefined depth may be greater than or less than the first predefined depth. Also, in some embodiments, the second wavelength may be greater than or less than the first wavelength.

In some embodiments, the first wavelength 106-1 may be less than the second wavelength 106-2 and the first predefined depth in the tissue 102 may be less than the second predefined depth in the tissue 102. Thus, in these embodiments, the light of the longer second wavelength 106-2 may be focused deeper in the tissue 102 than the light of the shorter first wavelength 106-1. In other embodiments, the first wavelength 106-1 may be less than the second wavelength 106-2 and the first predefined depth in the tissue 102 may be greater than the second predefined depth in the tissue 102. Thus, in these embodiments, the light of the shorter first wavelength 106-1 may be focused deeper than the light of the longer second wavelength 106-2.

In some embodiments, the first wavelength 106-1 may be greater than the second wavelength 106-2 and the first predefined depth in the tissue 102 may be less than the second predefined depth in the tissue 102. Thus, in these embodiments, the light of the shorter second wavelength 106-2 may be focused deeper in the tissue 102 than the light of the longer first wavelength 106-1. In other embodiments, the first wavelength 106-1 may be the greater than the second wavelength 106-2 and the first predefined depth in the tissue 102 may be greater than the second predefined depth in the tissue 102. Thus, in these embodiments, the light of the longer first wavelength 106-1 may be focused deeper than the light of the shorter second wavelength 106-2.

In this way, a spatial pattern of wavelength can be created in both a lateral direction and an axial direction. In some embodiments, the lens may comprise a single lens or a plurality (e.g. an array) of lenses. A single lens may have a chromatic aberration or an inverse chromatic aberration. A plurality of lenses (such as an array of lenses) may have a chromatic aberration and/or an inverse chromatic aberration.

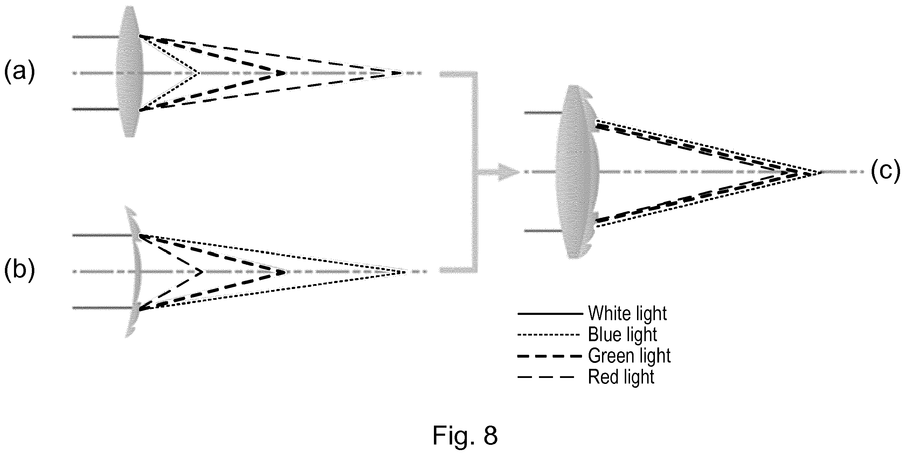

Figs. 8(a), (b) and (c) are simplified schematic illustrations of example lenses according to an embodiment. In more detail, Fig. 8(a) is a simplified schematic illustration of a refractive lens, Fig. 8(b) is a simplified schematic illustration of a phase Fresnel (PF) lens, and Fig. 8(c) is a simplified schematic illustration of a combined refractive and PF lens. For illustration purposes, the light source 104 of the device 100 is configured to emit white light through the example lenses. The light of blue wavelength, green wavelength, and red wavelength is shown following the emission of the white light through the example lenses.

The refractive lens illustrated in Fig. 8(a) has a chromatic aberration. As such, as illustrated in Fig. 8(a), the refractive lens is configured to focus longer wavelengths at greater depths than shorter wavelengths. That is, for the refractive lens, the longer the wavelength of light, the further the focal point is from the lens. For example, the red light is focused further away from the lens than the blue light and the green light, and the green light is focused further away from the lens than the blue light. The PF lens illustrated in Fig. 8(b) has an inverse chromatic aberration. As such, as illustrated in Fig. 8(b), the PF lens is configured to focus shorter wavelengths at greater depths than longer wavelengths. That is, for the PF lens, the shorter the wavelength of light, the further the focal point is from the lens. For example, the blue light is focused further away from the lens than the red light and the green light, and the green light is focused further away from the lens than the red light. As illustrated in Fig. 8(c), the combination of the refractive lens with the PF lens provides a mutual cancellation. The inverse chromatic aberration of the PF lens compensates for the chromatic aberration of the refractive lens.

Thus, a single lens with a chromatic aberration or an inverse chromatic aberration in an axial direction or a plurality of lenses (such as an array of lenses) with a chromatic aberration and/or an inverse chromatic aberration in an axial direction can be used to allow a deeper or shallower focusing of the first and second wavelengths 106-1, 106-2.

Although not illustrated in the figures, there is also provided a system comprising the device as described earlier. In some embodiments, the system may also comprise a processor. The processor can be implemented in numerous ways, with software and/or hardware, to perform the various functions described herein. In particular implementations, the processor can comprise a plurality of software and/or hardware modules, each configured to perform, or that are for performing, individual or multiple steps of the method described herein. The processor may comprise one or more processors (such as one or more microprocessors, one or more multi-core processors and/or one or more digital signal processors (DSPs)), one or more processing units, and/or one or more controllers (such as one or more microcontrollers) that may be configured or programmed (e.g. using software or computer program code) to perform the various functions described herein. The processor may be implemented as a combination of dedicated hardware (e.g. amplifiers, pre-amplifiers, analog-to-digital convertors (ADCs) and/or digital-to-analog convertors (DACs)) to perform some functions and a processor (e.g. one or more programmed microprocessors, DSPs and associated circuitry) to perform other functions.

In some embodiments, the processor can be configured to acquire an indication of a photoconversion state of the protein. In some embodiments, the processor can be configured to acquire the indication of the photoconversion state of the protein using optical spectroscopy. A person skilled in the art will be aware of various optical spectroscopy techniques and the manner in which the photoconversion state of the protein can be determined using these techniques. In some embodiments, the photoconversion state of the protein may be a ratio of the amount of protein to the amount of photoproduct protein. In some embodiments where the processor is configured to acquire an indication of a photoconversion state of the protein, the processor can also be configured to control the light source 104 of the device 100 to alternate between emitting the light of the first wavelength and the light of the second wavelength according to (or depending on) the photoconversion state of the protein. In this way, the effectiveness of the treatment can be further improved.

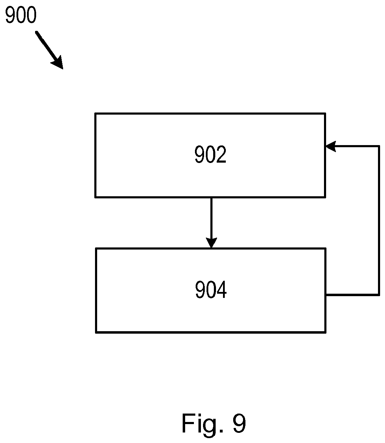

Fig. 9 is a flow chart illustrating a method 900 for treating tissue 102 according to an embodiment. More specifically, the method 900 of Fig. 9 is a method of operating the device 100 described earlier. The method 900 may generally be performed by or under the control of a processor, such as that described earlier.

With reference to Fig. 9, at block 902, light of a first wavelength is emitted to stimulate at least partial photoconversion of a protein in at least part of the tissue. At block 904 of Fig. 9, light of a second wavelength is emitted to at least partially reverse the at least partial photoconversion of the protein in the at least part of the tissue. The first wavelength and the second wavelength are different. As illustrated in Fig. 9, the method then proceeds again to block 902 of Fig. 9. That is, the method 900 of Fig. 9 comprises alternating between emitting the light of the first wavelength (block 902 of Fig. 9) and emitting light of the second wavelength (block 904 of Fig. 9).

More specifically, the method 900 of Fig. 9 comprises operating the light source 104 of the device 100 described earlier to alternate between emitting the light of the first wavelength and emitting light of the second wavelength. In some embodiments, a processor (such as that described earlier) may be configured to operate (e.g. control) the light source to alternate between emitting the light of the first wavelength and emitting light of the second wavelength. At least one or all of the steps of the method can be automated according to some embodiments.

There is also described a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein a device 100, a system, a method 900 and a computer program product that address the limitations associated with the existing techniques. Therefore, there is provided an improved device and system for treating tissue and an improved method for treating tissue. The device, system and method can be useful for reducing inflammation, improving blood flow, treating brain conditions related to blood flow, treating neurodegenerative diseases, treating inflammatory skin diseases, healing wounds, treating pigmentation disorders (e.g. vitiligo), improving anti-ageing, reducing blood pressure, improving hair growth, and similar. The device, system and method can be used to reduce collagen in tissue due to the anti-proliferative and anti-inflammatory action of the light. As such, the device, system and method can also be useful in preventing the formation of granulation tissue around an implanted device so that the implanted device is not deformed due to collagen contraction during the implantation period and/or in facilitating the extraction as less collagen is formed around the implant.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A device (100) for treating tissue (102), the device (100) comprising:
a light source (104) configured to emit light (106) to illuminate tissue (102), wherein the light source (104) is configured to alternate between:
emitting light of a first wavelength (106-1) to stimulate at least partial photoconversion of a protein in at least part of the tissue (102); and
emitting light of a second wavelength (106-2) to at least partially reverse the at least partial photoconversion of the protein in the at least part of the tissue (102), wherein the first wavelength and the second wavelength are different.

2. The device (100) as claimed in claim 1, wherein the light source (104) is configured to alternate between:
emitting the light of the first wavelength (106-1) to stimulate partial photoconversion of the protein in at least part of the tissue (102); and
emitting the light of the second wavelength (106-2) to at least partially reverse the partial photoconversion of the protein in the at least part of the tissue (102).

3. The device (100) as claimed in claim 2, wherein the light source (104) is configured to emit the light of the first wavelength (106-1) for a predefined time period to stimulate partial photoconversion of the protein in at least part of the tissue (102).

4. The device (100) as claimed in claim 2 or 3, wherein the light source (104) is configured to emit the light of the first wavelength (106-1) at a predefined intensity to stimulate partial photoconversion of the protein in at least part of the tissue (102).

5. The device (100) as claimed in claim 1, wherein the light source (104) is configured to alternate between:
emitting the light of the first wavelength (106-1) to stimulate photoconversion of the protein in a first part of the tissue (102); and
emitting the light of the second wavelength (106-2) to at least partially reverse the photoconversion of the protein in the first part of the tissue (102).

6. The device (100) as claimed in claim 5, wherein the light source (104) is configured to:
emit the light of the first wavelength (106-1) to illuminate the first part of the tissue (102) to stimulate photoconversion of the protein in the first part of the tissue (102); and
emit the light of the second wavelength (106-2) to illuminate a second part of the tissue (102) to at least partially reverse the photoconversion of the protein in the first part of the tissue (102).

7. The device (100) as claimed in claim 6, wherein the light source (104) comprises:
a first light source (104) configured to emit the light of the first wavelength (106-1) to illuminate the first part of the tissue (102); and
a second light source (104) configured to emit the light of the second wavelength (106-2) to illuminate the second part of the tissue (102).

8. The device (100) as claimed in claim 6, wherein the light source (104) comprises:
a filter configured to:
pass the light of the first wavelength (106-1) to illuminate the first part of the tissue (102); and
pass the light of the second wavelength (106-2) to illuminate the second part of the tissue (102).

9. The device (100) as claimed in claim 1, wherein the light source (104) is configured to alternate between:
emitting the light of the first wavelength (106-1) to stimulate photoconversion of the protein in a first part of the tissue (102) and emitting the light of the second wavelength (106-2) to at least partially reverse the photoconversion of the protein in the first part of the tissue (102); and
emitting the light of the first wavelength (106-1) to stimulate photoconversion of the protein in a second part of the tissue (102) and emitting the light of the second wavelength (106-2) to at least partially reverse the photoconversion of the protein in the second part of the tissue (102).

10. The device (100) as claimed in claim 9, wherein the light source (104) is configured to:
emit the light of the first wavelength (106-1) to illuminate the first part of the tissue (102) to stimulate photoconversion of the protein in the first part of the tissue (102); and
emit the light of the second wavelength (106-2) to illuminate the second part of the tissue (102) to at least partially reverse the photoconversion of the protein in the first part of the tissue (102).

11. The device (100) as claimed in claim 9 or 10, wherein the light source (104) is configured to:
emit the light of the first wavelength (106-1) to illuminate the second part of the tissue (102) to stimulate photoconversion of the protein in the second part of the tissue (102); and
emit the light of the second wavelength (106-2) to illuminate the first part of the tissue (102) to at least partially reverse the photoconversion of the protein in the second part of the tissue (102).

12. The device (100) as claimed in any of claims 6 to 11, wherein the device (100) comprises:
a lens configured to:
focus the light of the first wavelength (106-1) at a first predefined depth in the tissue (102); and/or
focus the light of the second wavelength (106-2) at a second predefined depth in the tissue (102), wherein the second predefined depth is the same as the first predefined depth or different to the first predefined depth.

13. A system comprising:
the device (100) as claimed in any of the preceding claims; and
a processor configured to:
acquire an indication of a photoconversion state of the protein; and
control the light source (104) to alternate between emitting the light of the first wavelength (106-1) and the light of the second wavelength (106-2) according to the photoconversion state of the protein.

14. A method (900) for treating tissue (102), the method (900) comprising:
alternating between:
emitting light of a first wavelength (106-1) to stimulate at least partial photoconversion of a protein in at least part of the tissue (102); and
emitting light of a second wavelength (106-2) to at least partially reverse the at least partial photoconversion of the protein in the at least part of the tissue (102), wherein the first wavelength and the second wavelength are different.

15. A computer program product comprising a computer readable medium, the computer readable medium having computer readable code embodied therein, the computer readable code being configured such that, on execution by a suitable computer or processor, the computer or processor is caused to perform the method as claimed in claim 14.
